# EUROPÄISCHE PATENTSCHRIFT

(11) **EP 3 270 045 B1**
(45) Veröffentlichungstag und Bekanntmachung des Hinweises auf die Patenterteilung: **07.06.2023**
(21) Anmeldenummer: 16178808.8
(22) Anmeldetag: 11.07.2016
(51) Int. Cl.: G01N 21/25, G01N 21/31, G01N 33/00, G01J 3/02, G01J 3/10, G01J 3/42

(54) **ANORDNUNG ZUM MESSEN VON GASKONZENTRATIONEN**
ASSEMBLY FOR THE MEASUREMENT OF GAS CONCENTRATIONS
SYSTEME DE MESURE DE CONCENTRATIONS DE GAZ

(43) Veröffentlichungstag der Anmeldung: 17.01.2018
(60) Teilanmeldung: 17184041.6
(73) Patentinhaber: Bluepoint Medical GmbH & Co. KG, 23923 Selmsdorf (DE)
(72) Erfinder: LINDNER, Bernd, 23167 Stockelsdorf (DE)
(74) Vertreter: Seemann & Partner Patentanwälte mbB

(56) Entgegenhaltungen:
- EP-A1- 1 398 617
- DE-A1-102011 116 367
- US-A1- 2010 252 737
- US-A1- 2013 110 311
- US-B2- 7 005 645
- RABE MARIAN ET AL: "Mode filter for LED-based absorption spectroscopy", 2016 IEEE INTERNATIONAL INSTRUMENTATION AND MEASUREMENT TECHNOLOGY CONFERENCE PROCEEDINGS, IEEE, 23. Mai 2016 (2016-05-23), Seiten 1-5, XP032928347, DOI: 10.1109/I2MTC.2016.7520591 [gefunden am 2016-07-22]
- DEGNER M ET AL: "Real time exhaust gas sensor with high resolution for onboard sensing of harmful components", 2008 IEEE SENSORS,LECCE, ITALY, IEEE, PISCATAWAY, NJ, USA, 26. Oktober 2008 (2008-10-26), Seiten 973-976, XP031375242, ISBN: 978-1-4244-2580-8
- DEGNER M ET AL: "LED-spectroscopy based on multi quantum well emitter", ELECTROMAGNETICS IN ADVANCED APPLICATIONS (ICEAA), 2012 INTERNATIONAL CONFERENCE ON, IEEE, 2. September 2012 (2012-09-02), Seiten 840-843, XP032247799, DOI: 10.1109/ICEAA.2012.6328751 ISBN: 978-1-4673-0333-0
- EBERT V ET AL: "SIMULTANEOUS DIODE-LASER-BASED IN SITU DETECTION OF MULTIPLE SPECIES AND TEMPERATURE IN A GAS-FIRED POWER PLANT", PROCEEDINGS OF THE COMBUSTION INSTITUTE, ELSEVIER, NL, no. 28, 1 January 2000 (2000-01-01), pages 423-430, XP001148198, ISSN: 1540-7489

## Beschreibung

Die Erfindung betrifft eine Anordnung zum Messen von Gaskonzentrationen in einem Gasabsorptionsverfahren, in welchem Licht von Lichtquellen verschiedener Wellenlängen im sichtbaren, UV- und/oder IR-Bereich, insbesondere LED-Lichtquellen, durch eine Messzelle mit einer zu analysierenden Gasmischung geleitet wird, insbesondere einer Mischung von NO₂, SO₂ und/oder NO in Luft, und Gaskonzentrationen zu messender Gase der Gasmischung über eine Messung einer Abschwächung des in die Messzelle eingeleiteten Lichts bei verschiedenen Wellenlängen aufgrund von Absorption in den verschiedenen Gasen der Gasmischung bestimmt werden.

Die Messung von Gaskonzentrationen mittels eines Gasabsorptionsverfahrens ist ein bekanntes Verfahren der Messtechnik. Es beruht auf der Eigenschaft von Gasen, Licht bestimmter Wellenlängen zu absorbieren und somit abzuschwächen. Das Maß der Abschwächung ist dann ein Maß für die Gaskonzentration des absorbierenden Gases. Die Messung der Abschwächung der Lichttransmission durch das Gas erfordert in der Regel eine Referenzmessung, aus der entweder direkt oder indirekt auf die Intensität des in das Gas eingestrahlten Lichts geschlossen werden kann.

Bekannte Möglichkeiten einer Referenzmessung beinhalten beispielsweise die Abzweigung eines Anteils des zunächst ausgestrahlten Lichts, das dann auf einen optischen Messempfänger geleitet wird, ohne das Messgas zu durchlaufen. Bei dieser direkten Messung wird allerdings nicht berücksichtigt, dass weitere optische Elemente, wie beispielsweise Lichteintrittsfenster und Lichtaustrittsfenster einer Messzelle oder Lichtleiter selbst eine absorbierende Wirkung entfalten und beispielsweise durch Verschmutzung intransparenter werden können.

Eine alternative Möglichkeit hierzu bietet sich darin, Absorptionsbandlücken in dem Absorptionsspektrum des zu messenden Gases oder der zu messenden Gase zu benutzen, um Licht mit einer Wellenlänge durch die Messzelle zu leiten, von dem bekannt ist, dass es nicht oder nur wenig in dem Messgas oder den Messgasen absorbiert wird. Dieses Licht erleidet die gleiche Abschwächung in den übrigen optischen Elementen einer solchen Messanordnung wie dasjenige Licht, das beispielsweise auf die Absorptionsmaxima der Messgase eingestellt ist. Eine durch zunehmende Verschmutzung der Messzelle beispielsweise wird auf diese Weise ausgeschlossen. Beachtlich ist hierbei, dass die Wellenlängen der Referenzmessung nicht zu unterschiedlich von den Wellenlängen der eigentlichen Absorptionsmessung sein sollten, weil ansonsten dispersive Effekte, also wellenlängenabhängige Effekte, eine störende Rolle spielen können.

Eine Anordnung, mit der ein entsprechendes Gasabsorptionsverfahren durchführbar ist, ist in der Patentanmeldung DE 10 2008 064 173 A1 der Anmelderin beschrieben. Die darin gezeigte Vorrichtung dient unter anderem zum Messen von Stickstoffmonoxid (NO), Stickstoffdioxid (NO₂), Schwefeldioxid (SO₂), Ozon (O₃) sowie Komponenten in fluiden Medien, für Verbrennungsmotoren, insbesondere bei der Online-Überwachung von Dieselmotoren, in der Umweltmesstechnik oder Medizintechnik, beispielsweise zur Messung der Atemluft. Darin werden verschiedenfarbige LEDs als Lichtquellen verwendet, deren Licht jeweils in eine Lichtleitfaser eingekoppelt wird.

Bei dieser Art der Lichterzeugung und Weiterleitung ist zu berücksichtigen, dass LEDs flächenhafte Lichterzeugungselemente sind und die Lichterzeugung nicht konstant über die gesamte Fläche der LEDs erfolgt. Vielmehr bilden sich zeitlich veränderliche Domänen auf den LEDs, die Licht emittieren, und die sich im Laufe der Zeit ändern, beispielsweise über die Oberfläche der LED wandern, sich teilen, erlöschen oder sich wieder vereinigen. Das Gleiche gilt für die Abstrahlungsrichtung, die zwar im Allgemeinen auf einen Abstrahlkegel innerhalb eines Abstrahlwinkels begrenzt ist, den Abstrahlkegel jedoch in zeitlich veränderlicher Weise bevölkert. Aus diesem Grund koppelt das von den LEDs erzeugte Licht in verschiedene Moden der Lichtleitfasern ein. Um zu verhindern, dass ausgangs der Lichtleitfasern Intensität und Abstrahlungsrichtung des aus den Lichtleitfasern austretenden Lichts fluktuiert, wird gemäß DE 10 2008 064 173 A1 eine Modenmischung vorgenommen, so dass das aus den Lichtleitfasern austretende Licht unabhängig geworden ist von dem ursprünglichen Ort und der ursprünglichen Richtung der Lichterzeugung.

Aus DE 10 2011 116 367 A1 ist eine alternative Anordnung bekannt, bei der eine sogenannte Multi Quantum Well-LED (MQW-LED) zum Einsatz kommt. Wie alle LEDs hat auch die MQW-LED ein Spektrum mit einer Breite von ca. 5 % bis 20 % der zentralen Wellenlänge, unterscheidet sich von herkömmlichen LEDs allerdings darin, dass die Form des Emissionsspektrums weitgehend temperaturunabhängig ist. Das Spektrum der MQW-LED wird gemäß DE 10 2011 116 367 A1 so gewählt, dass es bezüglich des Absorptionsspektrums eines zu messenden Gases Anteile hat, die eine starke Absorption erfahren, und andere Anteile, die eine geringe oder gar keine Absorption durch das zu messende Gas erfahren.

Ausgangs einer Messzelle wird das durchgestrahlte Licht der MQW-LED durch einen geeignet gewählten wellenlängenselektiven Strahlteiler in zwei Anteile aufgeteilt, bei denen in einem Referenzanteil wenig Absorption zu erwarten ist, und in einem anderen Anteil eine hohe Absorption entsprechend der Gaskonzentration des zu messenden Gases. Dies hat den Vorteil, dass aufgrund der engen Nachbarschaft der verschiedenen Frequenzanteile dispersive Effekte kaum auftreten und nur eine einzige Lichtquelle das Licht für die eigentliche Messung und für die Referenzmessung erzeugt. Voraussetzung hierfür ist die Temperaturstabilität des Emissionsspektrums, die bei herkömmlichen LEDs nicht gewährleistet ist.

Aus Rabe, Marian et al., "Mode Filter for LED-based absorption spectroscopy", 2016 IEEE International Instrumentation and Measurement Technology Conference Proceedings, IEEE, 23. Mai 2016, Seiten 1-5, ist eine Untersuchung zur Modenfilterung mittels in Form einer "8" geführten Glasfasern zur Vorbereitung einer Gasabsorptionsmessung mit mehreren Lichtquellen verschiedener Wellenlängen bekannt. Eine Erhöhung der Anzahl von Windungen über 7 bis 8 hinaus brachte dabei keine signifikante Verbesserung mehr. Die Tests wurden ohne tatsächliche Gasabsorptionsmessung ausgeführt.

In M. Degner et al., "Real time exhaust gas sensor with high resolution for onboard sensing of harmful components", 2008 IEEE Sensors, Lecce, Italien, IEEE, Piscataway, NJ, USA, Seiten 973 - 976, wird die Steuerung und Verringerung von Emissionen eines Verbrennungsmotors mithilfe eines optischen Sensorsystems auf der Grundlage von LEDs beschrieben, die im UV- und sichtbaren Bereich arbeiten. Die fasergekoppelten LEDs speisen ihr Licht in eine Messzelle ein, die von einem Messgas durchströmt wird. Nach Durchtritt und Absorption im Messgas wird das übrige Licht wiederum mittels Lichtleitfasern zu Fotodioden geführt. Die Lichtleitfasern entkoppeln die empfindlichen elektrooptischen Komponenten von der schädigenden Umgebung des Auspuffsystems.

Aus US 2010/0252737 A1 ist ferner ein Spektrometer zur Analyse von Fluiden bekannt, welches eine Beleuchtungsvorrichtung umfasst, die einen Lichtstrahl erzeugt, welcher ein Wellenlängenband abdeckt, eine Sonde, die so ausgebildet ist, dass der Lichtstrahl mit einem zu analysierenden Fluid wechselwirkt, und einer Spektralanalysevorrichtung, die das Licht nach seiner Wechselwirkung mit dem Fluid empfängt und Messungen vornimmt, die von der Menge an Licht bei verschiedenen Wellenlängenbereichen abhängen. Die Beleuchtungsvorrichtung enthält mehrere lichtemittierende Komponenten, die Licht in verschiedenen Wellenbereichen aussenden, und die über eine optische Komponente gemischt in einen gemeinsamen Lichtleiter eingekoppelt werden.

Aus EP 1 398 617 A1 ist ein Abgassensor bekannt, bei dem aus einem Abgasstrom eine Probe in eine Gasprobenzelle abgeleitet wird. Ein Emittermodul sendet Licht einer oder mehrerer Wellenlängen aus und koppelt diese über eine erste faseroptische Kopplung in die Gasmesszelle ein. Über eine zweite faseroptische Kopplung wird austretende Strahlung zu einem Detektionsmodul geführt. Es wird ein Ausgangssignal erzeugt, das die Intensität der nachgewiesenen Strahlung bei der einen oder mehreren Wellenlängen anzeigt, so dass die Gaskonzentration in der Gasprobe bestimmt wird. Temperaturvariationen in der Gasprobenzelle können ausgeglichen werden.

US 7,005,645 B2 D6 offenbart eine Vorrichtung und ein Verfahren zur simultanen Detektion von mehreren Gasspezies auf der Grundlage der Abschwächung von Laserlicht durch Absorptionen in einem Gasgemisch. Es werden N Laserquellen verwendet, die bei N unterschiedlichen Wellenlängen betrieben werden. Jede Laserquelle korrespondiert zu einer Gasspezies. Die Übertragung des Laserlichts in eine Messzelle erfolgt mit Faseroptik.

In Ebert, V., et al., "Simultaneous diode-laser-based in situ detection of multiple species and temperature in a gas-fired power plant", Proceedings of the Combustion Institute, Elsevier, NL, Nr. 28, 01.01.2000, Seiten 423 bis 430, wird eine gleichzeitige Detektion von verschiedenen Gastypen sowie einer Temperatur in einem Gaskraftwerk auf der Grundlage von Absorption von Licht offenbart, das von Diodenlasern bei verschiedenen Wellenlängen ausgesendet wird.

Aus DE 10 2011 116 367 A1 ist eine Vorrichtung zur Bestimmung der Konzentration einer oder mehrerer Substanzen in einem fluiden Medium bekannt, bei der die Lichtemission einer MQW-LED zur Absorptionsmessung benutzt wird. Dies wird auch in Degner, M., et al., "LED-spectroscopy based on multi quantum weil emitter", Electromagnetics in Advanced Applications (ICEAA), 2012 International Conference On, IEEE, 02.09.2012, Seiten 840-843 besprochen.

Demgegenüber liegt der vorliegenden Erfindung die Aufgabe zugrunde, eine kompakte Anordnung zur Gasabsorptionsmessung zur Verfügung zu stellen, mit der Gaskonzentrationen mehrerer Gase eines Gasgemisches auch unter extremen Umweltbedingungen mit hoher Genauigkeit und Geschwindigkeit messbar sind.

Diese Aufgabe wird durch eine Anordnung zum Messen von Gaskonzentrationen in einem Gasabsorptionsverfahren gemäß Patentanspruch 1 gelöst. Bevorzugte Ausführungsformen der Erfindung sind in den abhängigen Ansprüchen 2-14 definiert.

In einem der Erfindung zugrunde liegenden Gasabsorptionsverfahren wird Licht von Lichtquellen verschiedener Wellenlängen im sichtbaren, UV- und/oder IR-Bereich, insbesondere LED-Lichtquellen, durch eine Messzelle mit einer zu analysierenden Gasmischung geleitet, insbesondere einer Mischung von NO₂, SO₂ und/oder NO in Luft, und werden Gaskonzentrationen zu messender Gase der Gasmischung über eine Messung einer Abschwächung des in die Messzelle eingeleiteten Lichts bei verschiedenen Wellenlängen aufgrund von Absorption in den verschiedenen Gasen der Gasmischung bestimmt. Die erfindungsgemäße Anordnung umfasst mehrere Lichtquellen, deren unterschiedliche Wellenlängenspektren auf Absorptionsbänder, Absorptionslücken und/oder Übergangsbereiche zwischen Absorptionsbändern und Absorptionslücken der zu messenden Gase abgestimmt sind, eine Messzelle, wenigstens einen Messempfänger, mittels dessen eine Lichtintensität bei einer oder mehreren der eingestrahlten Wellenlängen ausgangs der Messzelle messbar ist, und eine Auswertevorrichtung, die ausgebildet ist, aus den gemessenen Lichtintensitäten die Gaskonzentrationen zu bestimmen, umfasst.

Die Erfindung beruht unter anderem darauf, dass bei der Anordnung die Messzelle einen längserstreckten schmalen Strahlengang mit einem eingangsseitigen Öffnungsdurchmesser *B* und einer Absorptionslänge *L* aufweist mit *L* > *B*, insbesondere *L* > *5*·*B*, insbesondere *L* > *10·B,* wobei die Messzelle einen Gaseinlass und einen Gasauslass aufweist, wobei eine Mehrzahl von Lichtquellen unterschiedlicher Wellenlängenspektren zu einer ersten Lichtquellengruppe zusammengefasst ist, wobei ein optischer Homogenisator zwischen die erste Lichtquellengruppe und die Messzelle zwischengeschaltet ist, wobei insbesondere der Homogenisator direkt oder über eine gemeinsame optische Baugruppe an die Lichtquellengruppe angekoppelt ist.

Unter einer direkten Ankopplung wird im Rahmen der vorliegenden Erfindung eine Ankopplung mit einem Luftspalt oder einem optischen Medium verstanden, beispielsweise einem optischen Kleber, gegebenenfalls auch mehreren optischen Klebepunkten. Eine geeignete gemeinsame optische Baugruppe kann beispielsweise eine Sammellinse oder Linsengruppe sein, die das Licht aller Lichtquellen der Lichtquellengruppe gemeinsam empfängt und auf den Homogenisator leitet. Auch ein Hohlspiegel oder ein vor der Lichtquellengruppe angeordnetes Multi-Linsen-Array ist ein solches geeignetes gemeinsames optisches Element. Nicht dazu gehören allerdings beispielsweise individuelle Lichtleitfasern, die jeweils nur an eine Lichtquelle der Lichtquellengruppe angekoppelt werden.

Der optische Homogenisator ist insbesondere ein Lichtleiter oder ist als solcher ausgebildet. Ferner ist insbesondere der optische Homogenisator stabförmig ausgebildet. Schließlich ist insbesondere vorgesehen, dass der optische Homogenisator ein stabförmiger Lichtleiter ist oder als solcher ausgebildet ist.

Die Erfindung beruht auf dem Grundgedanken, dass eine schnelle Messung schnell veränderlicher Gaskonzentrationen nur in sehr kleinen Messvolumina möglich ist, deren Gasinhalt auch sehr schnell ausgetauscht wird. Ansonsten würde ein langsamer Austausch des Messgases in der Messzelle dazu führen, dass schnelle Änderungen der Gaskonzentrationen ausgewaschen werden. Für eine hohe Genauigkeit ist allerdings prinzipbedingt eine große Absorptionslänge notwendig. Dies ist einerseits durch eine große Länge der Messzelle selbst und andererseits durch ein- oder mehrfache Spiegelung in der Messzelle realisierbar. Um gleichzeitig das Messvolumen klein zu halten, ist eine schmale bzw. schlanke längserstreckte Form des Strahlengangs in der Messzelle erfindungsgemäß gewählt worden. Die Form der Messzelle sollte an den Strahlengang möglichst gut angepasst sein, um so wenig wie möglich Totvolumen außerhalb des Strahlengangs aufzuweisen.

Ein weiterer entscheidender Faktor für eine hohe Messgenauigkeit liegt darin, dass eine möglichst große Lichtmenge durch die Messzelle zu einem Empfänger gebracht werden muss. Dabei ist es bei einer längserstreckten schmalen Messzelle eine Herausforderung, das von der Lichtquelle ausgesandte Licht überhaupt größtenteils in die Messzelle hinein zu bekommen. Um dies zu gewährleisten, wird zunächst die Mehrzahl der Lichtquellen, insbesondere LEDs, zu einer sehr kleinen und kompakten Lichtquellengruppe zusammengefasst. Diese bildet eine "mehrfarbige" kleine Lichtquelle. Die einzelnen Lichtquellen der Lichtquellengruppe sollten möglichst dicht beieinander angeordnet sein, um möglichst wenig Totfläche zwischen ihnen zu haben und somit ein hohes Verhältnis aus leuchtender zu nichtleuchtender Fläche zu haben.

Die Emissionsfläche der Lichtquellengruppe und der Abstrahlwinkel der Lichtquellengruppe definieren den zur Verfügung stehenden Phasenraum des ausgesandten Lichts, der möglichst vollständig in die Messzelle eingetragen werden sollte, um eine hohe Messgenauigkeit zu gewährleisten. Es ist zu beachten, dass der Strahlengang in der Messzelle einen sehr kleinen Phasenraum aufspannt. Die Lichtquellengruppe sollte dementsprechend eine kleine Gesamtemissionsfläche aufweisen, weil auf diese Weise von Beginn an nur ein kleiner Phasenraum bevölkert bzw. erzeugt wird. Ein Verlust an Lichtleistung und eine gewisse Selektion und Verkleinerung des Phasenraums erfolgt im Homogenisator, der hierdurch den vom durchgelassenen Licht eingenommenen Phasenraum an den Phasenraum des Strahlengangs der Messzelle anpasst, wobei gegebenenfalls noch optische Baugruppen aus Linsen oder Spiegel zum Einsatz kommen können. Das zur Verfügung stehende ursprünglich emittierte Licht wird auf diese Weise mit nur vergleichsweise geringen Verlusten in die Messzelle mit kleinem Strahlengangdurchmesser eingekoppelt wird. Der kleine Durchmesser ist somit förderlich für ein geringes Messvolumen und eine hohe zeitliche Auflösung der Messung, wobei gleichzeitig die Ausgangsleistung der Lichtquellen vergleichsweise gering gehalten werden kann.

Aufgrund der Zusammenstellung mehrerer einzelner Lichtquellen zu einer Lichtquellengruppe handelt es sich hierbei um einen nichtzusammenhängenden Phasenraum, so dass jeder Einschnitt durch Aperturen oder sonstige Hindernisse die Zusammenstellung und Verteilung der verschiedenen Wellenlängenspektren stark zulasten der Intensität einzelner Wellenlängen bzw. Lichtquellen beeinflussen kann. Da Gasabsorptionsmessungen bei Gasgemischen auch die Messungen der relativen Stärken des transportierten Lichts verschiedener Farben bzw. Wellenlängen beinhalten, würde eine solche selektive Beeinflussung die Ergebnisse verfälschen. Aus diesem Grund ist erfindungsgemäß ein Homogenisator vorhanden, der dafür sorgt, dass der zur Verfügung stehende Phasenraum, also die räumliche Verteilung und Winkelverteilung der einzelnen Lichtstrahlen, möglichst gleichmäßig von allen eingestrahlten Wellenlängen bevölkert wird, so dass unvermeidbare Einschnitte nicht selektiv zulasten einzelner Wellenlängen gehen. Dafür sammelt der Homogenisator das Licht der verschiedenen LEDs der Lichtquellengruppe, mischt ihre Herkunftsorte gleichmäßig und strahlt das Licht gleichmäßig und mit möglichst kleiner Apertur bzw. Eingangsöffnung ab, wobei die Lichtverluste der Übertragung von den Quellen zur Zielapertur bzw. Zielöffnung möglichst gering sein sollte. Solche Bauteile sind klein, robust und kostengünstig herzustellen und erlauben eine Miniaturisierung gegenüber den bisher verwendeten Glasfasern, die außerdem noch Faserkoppler und Modenmischer benötigen. Gegebenenfalls durch den Homogenisator erzeugte Intensitätsverluste sind gegenüber dem zuvor genannten Vorteil in Kauf zu nehmen.

Die Merkmalskombination des schlanken bzw. schmalen Strahlengangs in der Messzelle zusammen mit der auf einem kleinen Raum beziehungsweise einer kleinen Oberfläche zusammengefassten mehrfarbigen Lichtquellengruppe und dem dazwischen geschalteten Homogenisator erlaubt es nunmehr erstmals, die hohe Auflösung und Genauigkeit eines Gasabsorptionsmessverfahrens mit einer für eine kurze Messzeit notwendigen Miniaturisierung zu verknüpfen, ohne Kompromisse bei der Messgenauigkeit eingehen zu müssen.

Der Durchmesser der Lichtquellengruppe kann kleiner sein als eine Fläche des Öffnungsdurchmessers *B* des Strahlengangs der Messzelle, insbesondere weniger als ein Drittel der Fläche der Öffnung betragen. Der Homogenisator kann vorteilhafterweise ferner an der Seite der ersten Lichtquellengruppe eine Fläche aufweisen, die einer Fläche der ersten Lichtquellengruppe im Wesentlichen entspricht, insbesondere nicht mehr als 40% von der Fläche der ersten Lichtquellengruppe abweicht. Diese Ausführung erlaubt eine besonders starke Miniaturisierung, da der Homogenisator mit den entsprechenden Größenverhältnissen direkt auf die Lichtquellengruppe aufgesetzt werden kann und das ausgestrahlte Licht zu einem gro-βen Teil weiterleiten kann. Eine etwas weniger miniaturisierte Ausführungsform wird erreicht, wenn die Eintrittsfläche des Homogenisators deutlich größer als die Fläche der ersten Lichtquellengruppe ist, insbesondere mehr als doppelt so groß. Diese Ausführungsform ist dann vorteilhaft, wenn baubedingt eine Beabstandung zwischen Lichtquellengruppe und Homogenisator notwendig ist.

Ein ausgangsseitiger Durchmesser *D*₂ des Homogenisators an der Seite der Messzelle ist vorzugsweise kleiner als der Öffnungsdurchmesser *B* des Strahlengangs der Messzelle.

Bei der Auswertevorrichtung handelt es sich vorzugsweise um eine programmierbare Datenverarbeitungsanlage mit Signaleingängen und Signalverarbeitungseinheiten oder um einen digitalen Signalprozessor (DSP) oder Mikrocontroller mit vorgeschalteten Analog-Digitalwandlern oder um andere zu Messzwecken üblicherweise verwendete Analysatoren.

Mit der erfindungsgemäßen Anordnung sind Messgenauigkeiten im ppm-Bereich und kleiner mit Messwiederholungsraten von 10/s bis 100/s erzielbar.

In einer vorteilhaften Weiterbildung umfasst die erste Lichtquellengruppe LED-Lichtquellen mit einem charakteristischen Abstrahlwinkel und ist die LED-Lichtquellengruppe so vor dem Homogenisator angeordnet, dass ein Abstrahlkegel der LED-Lichtquellen der ersten Lichtquellengruppe, gegebenenfalls nach Durchtritt durch die gemeinsame optische Baugruppe, im Wesentlichen vollständig in den Homogenisator eintritt. Der Abstrahlkegel umfasst einen Großteil des ausgestrahlten Lichts und umfasst nach einer Definition den Winkel, der von den seitlichen Punkten mit halber Maximal-Lichtstärke eingeschlossen wird. Mit dieser Maßnahme wird sichergestellt, dass Intensitätsverluste beim Eintritt in den Homogenisator so gering wie möglich gehalten werden. Dies trägt dazu bei, eine hohe Messgenauigkeit zu erreichen.

Vorzugsweise ist der Homogenisator als ein geformter transparenter massiver Lichtleiter auf der Grundlage von Totalreflexion an der Oberfläche oder von Brechzahlgradienten im Substrat oder als hohle Reflektoranordnung mit einem transparenten Medium im Inneren, insbesondere einem transparenten Gas oder Vakuum, und verspiegelten seitlichen Begrenzungsflächen ausgebildet, wobei der Homogenisator linear oder gebogen mit kreisrundem, ovalem oder polygonalem Querschnitt ausgebildet ist. Insbesondere ist der Homogenisator im Querschnitt sechseckig. Durch Mehrfachreflexion oder -beugung bevölkert sich der zur Verfügung stehende Phasenraum in diesen Homogenisatoren zunehmend gleichmäßig. Eine weitere Verdichtung der Belegung des Phasenraumes ergibt sich, wenn sich vorteilhafterweise der Homogenisator im Querschnitt in Richtung zur Messzelle hin verändert, insbesondere verjüngt.

Die Homogenisierung wird vorzugsweise weiter dadurch verbessert, dass Störstellen im oder am Homogenisator angeordnet sind, insbesondere Fehlstellen im Substrat, Streukörper in einem Spiegelhohlraum oder Rauigkeiten an Grenzflächen oder Spiegelflächen. Solche Störstellen führen zu einer starken Streuung des Lichts. Die dadurch erreichte starke Homogenisierung wird mit einem höheren Verlust an Lichtintensität durch Streuung aus dem Homogenisator heraus erkauft, jedoch kann der Vorteil der deutlich stärkeren Homogenisierung des einfallenden Lichts den Verlust an zur Verfügung stehender Lichtintensität mehr als wettmachen.

Die Messzelle weist, vorzugsweise, an ihrer Eingangsöffnung und/oder ein an die Messzellen angeordnetes Element der Anordnung weist ein kombiniertes Lichteintritts- und -austrittsfenster und eine gegenüber dem Lichteintritts- und -austrittsfenster liegende lichtreflektierende Wand auf oder, alternativ, jeweils ein Lichteintrittsfenster und ein Lichtaustrittsfenster, mit oder ohne spiegelnde Wände zwischen dem Lichteintrittsfenster und dem Lichtaustrittsfenster, wobei das oder die Lichteintrittsfenster und/oder Lichtaustrittsfenster insbesondere gegenüber einer Längserstreckung der Messzelle schräg gestellt ist oder sind. Der Fall des kombinierten Lichteintritts- und -austrittsfensters mit der lichtreflektierenden, also spiegelnden, Wand beschreibt den Fall einer Reflexionszelle, der andere Fall den einer Transmissionszelle.

Der Abschluss der Messzelle durch Fenster ermöglicht eine Abtrennung der Messzelle vom restlichen optischen Messaufbau und somit deren Austausch, Reinigung, Wartung und Ersatz. Eine Schrägstellung der Fenster verringert störende Reflexionen und ist mechanisch günstig zum Einfügen in einen Aufbau. Ebenfalls werden strömungstechnische Totvolumina etwas verringert. Die Messzelle kann auch als Ganzes aus dem Aufbau herausgenommen werden, sodass ein einfacher Austausch gegebenenfalls zum Zwecke der Wartung oder Reinigung möglich ist.

Gemäß einer weiteren Ausführungsform ist daher vorgesehen, dass die Messzelle herausnehmbar ist. Insbesondere ist die Messzelle werkzeuglos demontierbar.

Zu Kalibrierzwecken kann die Messzelle vorteilhaft durch eine Kalibrierzelle mit definierter wellenlängenabhängiger Absorption ersetzt werden, was teure Kalibriergase einspart. Falls das oder die Fenster nicht an der Messzelle selbst, sondern an einem angrenzenden Element der Anordnung angeordnet sind, besteht die Messzelle aus einer Röhre, die mittels einer Dichtung gasdicht gegenüber dem Fenster oder den Fenstern eingesetzt wird. Diese Variante hat den Vorteil, dass die Fenster gleich in der Anordnung gereinigt werden können und die restliche Anordnung vor Reinigungsflüssigkeit schützen. Es ergibt sich hierdurch eine modulare, insbesondere hermetische, Anordnung.

Vorzugsweise sind der Gaseinlass und der Gasauslass parallel zum Durchmesser der Messzelle versetzt angeordnet, um einen raschen Gasaustausch zu ermöglichen.

Wenn vorteilhafterweise eingangs und/oder ausgangs der Messzelle ein oder mehrere insbesondere wellenlängenselektive Strahlteiler angeordnet ist oder sind, mit dem oder mit denen Licht unterschiedlicher Lichtquellen der ersten Lichtquellengruppe und/oder einer zweiten Lichtquellengruppe zu zwei oder mehr verschiedenen Messempfängern geleitet wird, ist es möglich, die verschiedenen Wellenlängen, die die verschiedenen Lichtquellen der Lichtquellengruppe ausgestrahlt haben, und die auf Absorptionsbänder und Absorptionslücken verschiedener Messgase ausgelegt sind, wieder voneinander zu trennen und unabhängig voneinander die Abschwächung bei diesen Wellenlängen zu messen.

Die der Erfindung zugrunde liegende Aufgabe betrifft im Ganzen eine Anordnung zum Messen von Gaskonzentrationen in einem Gasabsorptionsverfahren gemäß Anspruch 1.

Die zweite Lichtquellengruppe kann eine einzelne Lichtquelle umfassen, womit der Anordnung eine weitere Wellenlänge für ein weiteres Messgas der Gasmischung hinzugefügt wird. Die zweite Lichtquellengruppe kann auch mehrere unterschiedliche Lichtquellen umfassen.

Grundsätzlich hat die erfindungsgemäße Anordnung den Vorteil, dass mehrere Gase in ein und demselben Messvolumen gemessen werden können, was die Messgenauigkeit erhöht, da räumliche und dynamische Fehler bei der Gasaufteilung auf mehrere Messzellen vermieden werden. Vorzugsweise überlappen hierzu die beiden Strahlwege in einem Messvolumen der Messzelle teilweise oder ganz.

Für die Messung mehrerer Gase mittels LED-Spektroskopie werden mehrere Lichtquellen benötigt, die aus unterschiedlichen Richtungen in die Messzelle angekoppelt werden können, um so ein zeitlich paralleles Messen mit geringem Übersprechen zu ermöglichen. Dies bietet sich besonders auch bei der Kombination unterschiedlicher Messverfahren an, wie sie beispielsweise aus DE 10 2008 064 173 A1 und DE 10 2011 116 367 A1 bekannt sind. Anstelle der teuren und großen Glasfasern, die in DE 10 2008 064 173 A1 verwendet werden, kommt eine Miniaturisierung zum Einsatz, da die benötigten LEDs sehr eng nebeneinander positioniert werden und das emittierte Licht nur von einem Homogenisator aufgefangen und homogenisiert wird und dann über einen externen Strahlteiler, beispielsweise einen halbdurchlässigen Spiegel, in einen Pfad zur Gasmessung und einen Referenzpfad aufgeteilt wird.

Da, erfindungsgemäß, die beiden Strahlwege gegenläufig durch die Messzelle führen, sind die Messungen mit dem ersten Strahlweg und dem zweiten Strahlweg ideal entkoppelt, was besonders vorteilhaft ist, wenn die verwendeten Wellenlängen der Lichtquellen nahe beieinander liegen. Es gibt aber auch andere Möglichkeiten, die Strahlwege voneinander im Wesentlichen zu entkoppeln, beispielsweise die Definition verschiedener Bereiche der Messzelle, die von den einzelnen Strahlwegen bestrahlt werden oder von Winkeln, unter denen die Strahlwege in die Messzelle eintreten.

In einer bevorzugten Weiterbildung sind beide Lichtquellengruppen zusammen mit dem jeweils dazu gehörenden Strahlweg und der Messzelle als zuvor beschriebene erfindungsgemäße Anordnungen ausgebildet, wobei die Messzelle beiden Anordnungen gemeinsam ist. Alternativ und ebenfalls bevorzugt umfasst eine bzw. die zweite Lichtquellengruppe wenigstens eine MQW-LED mit temperaturstabilem Emissionsspektrum und der zur zweiten Lichtquellengruppe gehörende Strahlweg ausgangs der Messzelle einen wellenlängenselektiven Strahlteiler und zwei Messempfänger, wobei der wellenlängenselektive Strahlteiler eingerichtet ist, das Emissionsspektrum der MQW-LED in zwei oder mehr Anteile aufzuspalten und die Anteile getrennt voneinander zu den beiden Messempfängern zu leiten.

Im letzteren Fall entspricht die Teilmessanordnung mit der MQW-LED im Wesentlichen derjenigen, die aus DE 10 2011 116 367 A1 der Anmelderin bekannt ist. Diese Version ist besonders gut für Gase mit schmalbandigen Absorptionsspektren geeignet wie beispielsweise Stickstoffmonoxid (NO). Solche Filter können Kantenfilter oder Bandpassfilter sein.

Im Kontext der vorliegenden Beschreibung wird unter einem "wellenlängenselektiven Strahlteiler" sowohl ein einzelner Strahlteiler, der die Strahlteilung wellenlängenselektiv vornimmt, als auch eine Gruppe von optischen Bauelementen verstanden. Eine solche Gruppe von optischen Bauelementen umfasst beispielsweise einen Strahlteiler, welcher nicht wellenlängenselektiv ist, und zumindest zwei Filter. In den geteilten Strahlengängen ist in Lichteinfallsrichtung auf den Strahlteiler folgend jeweils einer der Filter vorgesehen. Bei den Filtern handelt es sich beispielsweise um Kanten- oder Bandpassfilter, welche die Funktionalität der Wellenlängenselektion bereitstellen.

Das Emissionsspektrum der wenigstens einen MQW-LED sowie eine Wellenlängencharakteristik des wellenlängenselektiven Strahlteilers sind vorzugsweise so auf ein Absorptionsspektrum eines zu messenden Gases abgestimmt, dass ein erster Anteil des Emissionsspektrums der MQW-LED eine höhere Absorption im Gas erfährt als ein zweiter Anteil. Damit ist das Licht der einen MQW-LED in einen Messanteil und einen Referenzanteil aufgespalten. Systematische Effekte werden so auf ideale Weise unterdrückt. Auch, wenn der Referenzanteil nicht vollständig ohne Absorption ist, so sind doch diese Emissionsspektren der MQW-LED und die Gasabsorptionsspektren der zu messenden Gase bekannt, sodass durch die Messung im Messanteil und im Referenzanteil auf den Fall der idealen Nichtabsorption extrapoliert werden kann, woraus sich die zu bestimmende Gaskonzentration dann ergibt.

Wenn vorzugsweise die Anordnung eine Druck- und/oder Temperaturmesseinrichtung umfasst, die mit der Messzelle zur Messung eines Drucks und/oder einer Temperatur des Gasgemisches in der Messzelle verbunden ist, wobei die Auswertevorrichtung ausgebildet ist, den Einfluss eines gemessenen Druckniveaus oder von Druckschwankungen und/oder der Temperatur oder Temperaturschwankungen auf die Lichtabsorption oder die Gaskonzentrationen bei der Bestimmung der Gaskonzentrationen zu berücksichtigen, gegebenenfalls auf einen Normaldruck und/oder eine Normaltemperatur zu extrapolieren, dann ist es möglich, solche Umwelteinflüsse bei der Bestimmung der Gaskonzentrationen zu berücksichtigen, die einen eigenen Einfluss auf die Lichtabschwächung haben. Diese sind in besonderem Maße der Druck des Gasgemisches und die Temperatur.

In einer vorteilhaften Ausgestaltung wird Messgas in einem Nebenstromverfahren aus einem Hauptgasstrom in die Messzelle geleitet und nach Durchlaufen der Messzelle wieder in den Hauptgasstrom eingeleitet oder in die Umgebungsluft entlassen. Dieses Nebenstromverfahren erlaubt es, die Anordnung in einem größeren Abstand von einer für die Lichtquellen und Messempfänger unzuträglichen Umgebung anzuordnen. Dies ist beispielsweise in den Motoren von Kraftfahrzeugen, insbesondere Dieselmotoren, der Fall.

Falls eine weitere Beabstandung der Lichtquellen oder der Messempfänger von der Messzelle gewünscht ist, erfolgt vorzugsweise eine Lichteinkopplung in die Messzelle und/oder eine Lichtauskopplung aus der Messzelle unter Verwendung zusätzlicher Lichtleiter. Falls das Messvolumen direkt in einer unzuträglichen Umgebung liegen soll, ist eine zusätzliche Erhöhung des Abstandes der Messempfänger, Lichtquellen und Elektroniken von der Messzelle gewünscht. Hierzu erfolgt vorzugsweise die Lichteinkopplung in die Messzelle und/oder die Lichtauskopplung aus der Messzelle unter Verwendung zusätzlicher Lichtleiter.

Weitere Merkmale der Erfindung werden aus der Beschreibung erfindungsgemäßer bevorzugter Ausführungsformen zusammen mit den Ansprüchen und den beigefügten Zeichnungen ersichtlich. Erfindungsgemäße Ausführungsformen können, innerhalb des Schutzbereichs der Ansprüche, einzelne Merkmale oder eine Kombination mehrerer Merkmale erfüllen.

Die Erfindung wird nachstehend ohne Beschränkung des mittels der Patentansprüche definierten allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1a), b): Emissions- und Absorptionsspektren von Messgasen und LEDs
- Fig. 2: eine schematische Präsentation einer Anordung gemäß eines Ausführungsbeispiels der Erfindung,

- Fig. 3: Beispiele erfindungsgemäßer Homogenisatoren,
- Fig. 4: eine schematische Darstellung eines Teils einer Anordung gemäß eines Ausführungsbeispiels der Erfindung,
- Fig. 5: eine schematische Darstellung einer Homogenisierung ohne Streuzentren,
- Fig. 6: eine schematische Darstellung einer Homogenisierung mit Streuzentren,
- Fig. 7a)-d): schematische Darstellungen von Lichtleitungsprinzipien von Homogenisatoren gemäß Ausführungsbeispiele der Erfindung und
- Fig. 8: das spektrale Überlappungs- und Aufteilungsprinzip bei der Verwendung von MQW-LED ist zur Gasabsorptionsmessung.

In den Zeichnungen sind jeweils gleiche oder gleichartige Elemente und/oder Teile mit denselben Bezugsziffern versehen, so dass von einer erneuten Vorstellung jeweils abgesehen wird.

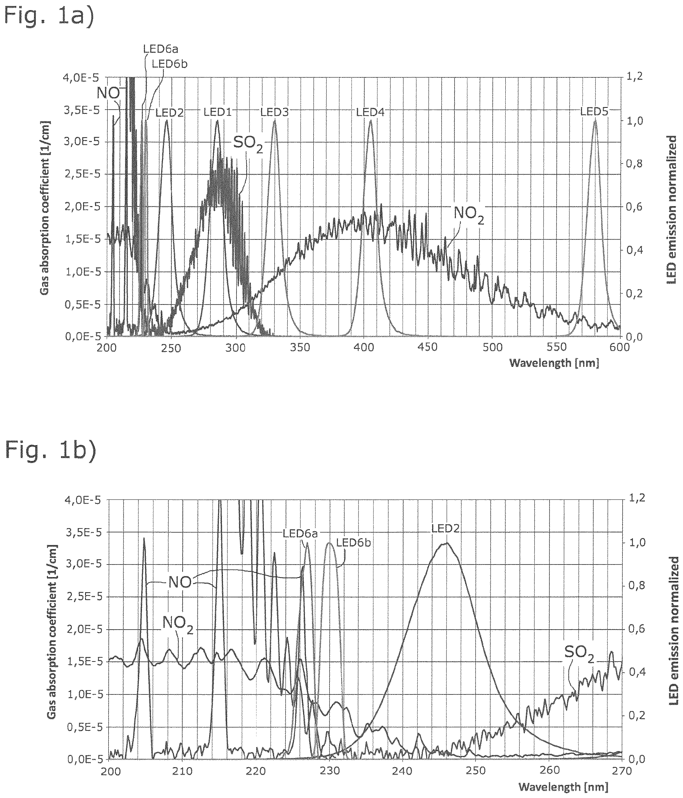

In den Figuren 1a) und 1b) sind jeweils die Absorptionsspektren der Gase Stickstoffmonoxid (NO), Schwefeldioxid (SO₂) und Stickstoffdioxid (NO₂) dargestellt, zusammen mit den Emissionsspektren von sechs verschiedenen LEDs, die willkürlich auf eine gemeinsame Maximalemission normalisiert sind. Ein solches Gasgemisch kommt beispielsweise im Abgas von Kraftfahrzeugmotoren vor, wobei die Verhältnisse von NO und NO₂ von der Temperatur abhängen. Die Wellenlängen beginnen bei 200 nm, also im UV-Bereich, und gehen in Figur 1a) bis 600 nm. Der Ausschnitt in Figur 1b) befindet sich vollständig im UV-Bereich zwischen 200 nm und 270 nm.

Das Absorptionsspektrum von NO₂ ist besonders breitbandig und hat ein Maximum bei ca. 400 nm. Sowohl die LED3 bei 330 nm als auch die LED4 bei ca. 405 nm erfahren eine signifikante Absorption in NO₂. Die längerwellige LED5 bei 580 nm kann als Referenz dienen, da sie sehr viel weniger Absorption in NO₂ erfährt. Im UV-Bereich befindet sich das schmalere Absetzungsspektrum von SO₂ mit einer Breite von ca. 60 nm um das Maximum bei 285 nm. Die LED1 ist mit ihrem Emissionsspektrum hierauf zentriert. Eine weitere LED2 ist mit ihrem Emissionsspektrum um ein Maximum bei ca. 246 nm zentriert und liegt in einem lokalen Minimum der Absorptionsspektren aller drei dargestellter Gase. Damit eignet sich diese Wellenlänge der LED 2 als Referenzwellenlänge und hat den weiteren Vorteil beispielsweise gegenüber LED5, dass neben einer geringen Absorption auch die dispersiven Effekte gegenüber den Wellenlängen von beispielsweise LED1 und LEDS, auch LED 4, geringer sind.

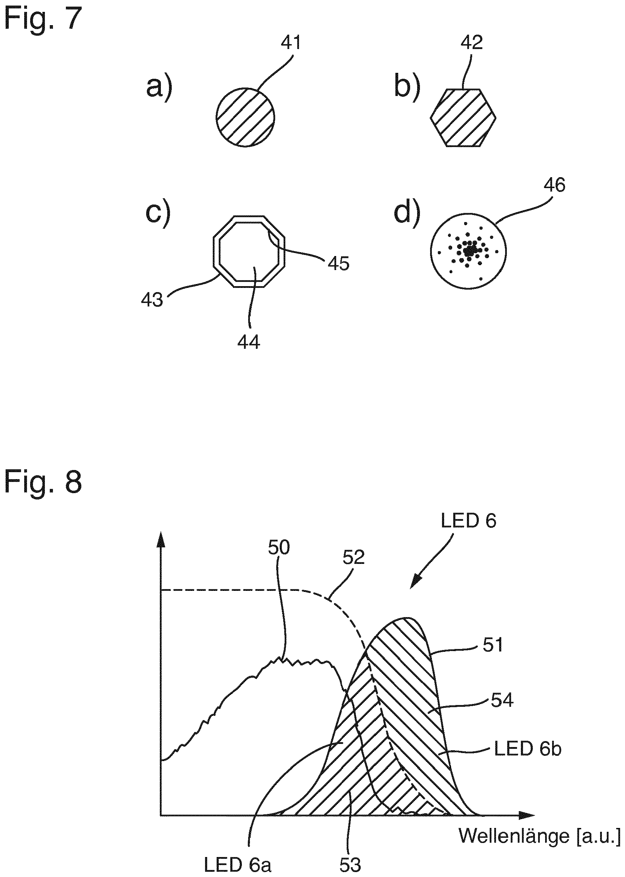

Wie besonders gut in Figur 1b) zu erkennen ist, besteht das Absorptionsspektrum von NO aus mehreren schmalen Bändern mit Breiten von wenigen Nanometern bei 205 nm, 215 nm und 226 nm. Eine MQW-LED mit der Bezeichnung LED6 erzeugt Licht in einem Bereich mit einer Breite von ca. 8 nm um 248 nm herum. Das Spektrum ist aufgeteilt in zwei Bereiche mit den Bezeichnungen LED6a und LED6b, die durch Aufspaltung in einem wellenlängenselektiven interferometrischen Strahlteiler mit steiler Flanke erzeugt werden. Dabei hat der kürzerwellige Anteil LED6a einen großen Überlapp mit dem Absorptionspeak von NO bei 226 nm, während der längerwellige Anteil LED6b kaum einen Überlapp mit dem Absorptionspeak hat und somit als Referenz aus derselben Lichtquelle verwendet werden kann. Das Prinzip ist unten in Figur 8 noch einmal deutlicher erläutert.

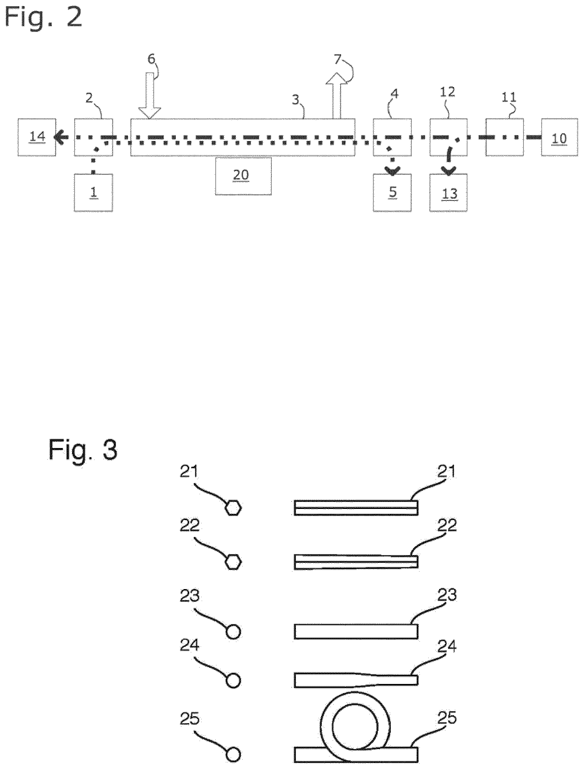

Figur 2 zeigt eine schematische Darstellung einer Anordnung gemäß eines Ausführungsbeispiels der Erfindung, in deren Zentrum eine Messzelle 3 mit einem Messgas bzw. Gasgemisch steht. Bei der Messzelle 3 handelt es sich um eine schlanke, langgestreckte, beispielsweise zylindrische Messzelle 3, was den Vorteil hat, dass bei geringem Volumen und somit möglicher hoher Austauschrate des Messgases eine große Absorptionslänge erreichbar ist. Hierfür verfügt die Messzelle 3 über einen Gaseinlass 6 und einen Gasauslass 7.

Auf der rechten Seite ist eine Lichtquellengruppe 10 dargestellt, in der mehrere verschiedenfarbige Lichtquellen, beispielsweise LEDs, erfindungsgemäß auf kleinem Raum zusammengefasst sind. Deren ausgestrahltes Licht gelangt durch einen Homogenisator 11, der im Folgenden noch näher erläutert wird, und über Strahlteiler 12, 4 zur Messzelle 3. Der Strahlteiler 12 kann die Funktion erfüllen, einen Teil des eingestrahlten Lichts als Referenz zu einem Referenz-Messempfänger 13 zu leiten. Dazu kann der Strahlteiler 12 wellenlängenselektiv sein, muss es aber nicht. Der restliche Anteil des Lichts aus der Lichtquellengruppe 10 tritt durch die gesamte Länge der Messzelle 3 hindurch, erfährt dabei eine wellenlängenabhängige Absorption im Gasgemisch und gelangt am anderen Ende zum Messempfänger 14, der ausgebildet ist, die Intensität des auf ihn fallenden Lichts zu messen. Durch Vergleich mit einem Sollwert oder durch Vergleich mit der Intensität im Referenz-Messempfänger 13 wird dann die Abschwächung berechnet oder ermittelt und daraus die ansprechenden Gaskonzentrationen der zu untersuchenden Gase bestimmt.

Mit der Messzelle 3 ist eine Druck- und/oder Temperaturmesseinrichtung 20 verbunden, die Druck und oder Temperatur des Gases in der Messzelle 3 misst und an die nicht dargestellte Auswertevorrichtung weiterleitet, die hieraus Korrekturen an der Bestimmung der Gaskonzentrationen vornehmen kann.

Die Anordnung gemäß Figur 2 weist einen zweiten Messpfad auf, der dem ersten, zuvor beschriebenen Messpfad entgegengesetzt ausgerichtet ist. Der zweite Messpfad beginnt mit einer Lichtquellengruppe 1, die im einfachsten Fall eine einzelne im MQW-LED umfasst oder aufweist. Es können auch mehrere MQW-LEDs umfasst sein oder Mischungen verschiedener LEDs mit oder ohne MQW-LED. In dem gezeigten Ausführungsbeispiel ist wenigstens eine MQW-LED umfasst, beispielsweise die LED6 der Figur 1. Ein Strahlteiler 2 lenkt das Licht der Lichtquellengruppe 1, also der MQW-LED, in die Messzelle 3 ein und trennt die Strahlwege des ersten Messpfads und des zweiten Messpfads voneinander. Nach Durchlaufen der Messzelle trennt ein Strahlteiler 4, der ein wellenlängenselektiver Strahlteiler sein kann, wiederum die beiden Messpfade bzw. Strahlwege voneinander und lenkt das Licht aus der Lichtquellengruppe 1 in eine Messanordnung mit einem Messempfänger 5. Diese Messanordnung kann einen weiteren Strahlteiler umfassen, der eine Aufteilung mit einer steilen Flanke entsprechend der obigen Beschreibung und der Figur 8 hierunter vornimmt. In diesem Fall sind zwei optische Messempfänger, beispielsweise Fotodioden, im Einsatz.

Anstelle zweier Strahlteiler 4, 12 kann auch ein gemeinsamer Strahlteiler verwendet werden, der entsprechend ausgebildet ist, beide Strahlwege wellenlängenselektiv voneinander zu entkoppeln, wobei er für die Wellenlängen der ersten Lichtquellengruppe 10 teiltransparent und teilreflektierend sein sollte. Die Messempfänger 5 und 13 sind in einem solchen Fall an gegenüberliegenden Seiten des Hauptstrahlengangs angeordnet.

In Figur 3 sind verschiedene Beispiele von Homogenisatoren 21 bis 25 schematisch dargestellt. Der Homogenisator 21, der auf der linken Seite im Querschnitt und auf der rechten Seite von der Seite dargestellt ist, ist ein herkömmlicher massiver Lichtleiter mit 6-eckigen bzw. hexagonalem Querschnitt. Der Homogenisator 22 unterscheidet sich hiervon darin, dass er sich vom Eingang zum Ausgang hin verjüngt, was zu einer stärkeren Homogenisierung beiträgt. Solche hexagonale Querschnitte haben die Eigenschaft, dass an den Stoßkanten zwischen 2 glatten Flächen vermehrt Lichtverluste auftreten, während sie bei runden Querschnitten stärker über den gesamten Umfang verteilt sind.

Der Homogenisator 23 hat im Gegensatz zu dem Homogenisator 21 einen runden Querschnitt. Der Homogenisator 24 hat ebenfalls einen runden Querschnitt, verjüngt sich allerdings. Der Homogenisator 24 schließlich hat einen runden Querschnitt und einen konstanten Durchmesser, beschreibt allerdings eine Biegung um 360°, was zu einer starken Homogenisierung führt. In allen Fällen handelt es sich um massive Homogenisatoren, beispielsweise aus Glas oder PLEXIGLAS^{®}.

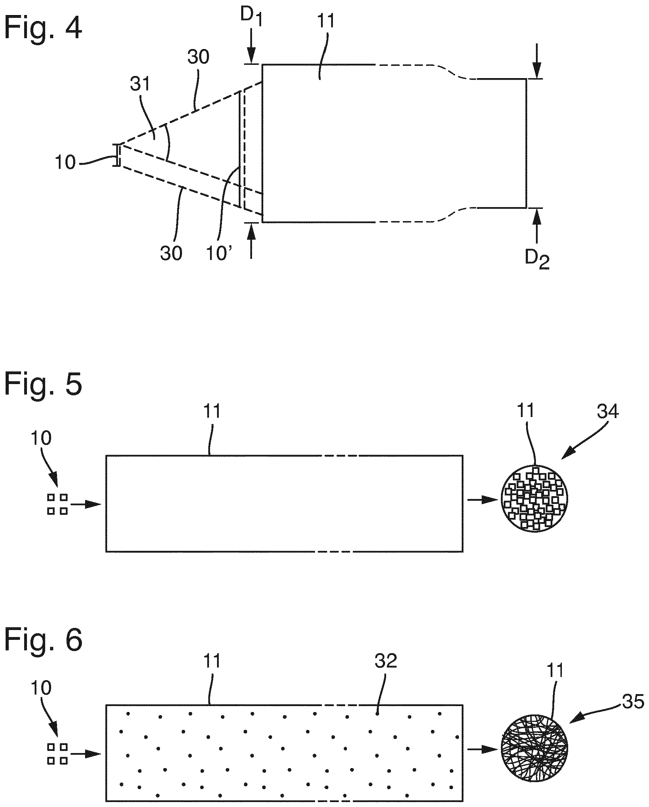

In Figur 4 ist schematisch die Kombination aus Lichtquellengruppe 10 und Homogenisator 11 dargestellt. Die Lichtquellengruppe 10 verfügt über mehrere als kurze Striche angedeutete Einzel-LEDs verschiedener Wellenlängen bzw. Farben, die auf einem kleinen Raum aneinander gesetzt sind. Dabei wird es sich um einen Durchmesser dieser Lichtquellengruppe 10 von üblicherweise weniger als 1 bis 2 mm handeln. Wie die meisten LEDs haben diese LEDs einen Abstrahlwinkel 31, was zu einem vollständigen Abstrahlkegel 30 der Lichtquellengruppe 10 führt, der von der Gesamtfläche der Lichtquellengruppe 10 ausgeht und sich zur Eintrittsfläche des Homogenisators 11 hin ausweitet. Der Abstand zwischen der Lichtquellengruppe 10 und der Eingangsfläche des Homogenisators 11 ist so gewählt, dass der Kegel 30 bei Eintritt in den Homogenisator 11 einen Durchmesser aufweist, der kleiner ist als die Öffnungsapertur *D₁* des Homogenisators 11. Im Inneren des Homogenisators, der sich zum Ausgang hin zu einem kleineren Durchmesser *D₂* verjüngt, findet eine Lichtleitung mit keinem oder wenigem Intensitätsverlust statt.

Den gleichen Abstrahlkegel 30 spannt auch eine im Verhältnis grö-βere Lichtquellengruppe 10' auf, die umso näher am Homogenisator 11 angeordnet ist. Zum Zwecke stärkerer Miniaturisierung ist die Wahl günstiger, den Homogenisator 11 in etwa so groß zu machen wie die Lichtquellengruppe 10' und ihn entsprechend dicht vor die Lichtquellengruppe 10' zu platzieren.

In den Figuren 5 und 6 ist jeweils dargestellt, wie der Phasenraum, in diesem Fall die Belegung der räumlichen Verteilung, durch den jeweiligen Homogenisator 11 vergleichmäßigt wird. Die räumliche Verteilung des ausgestrahlten Lichtes ist zunächst die räumliche Anordnung der LEDs der Lichtquellengruppe 10, in den Figuren 5 und 6 jeweils links dargestellt. Nach Durchgang durch den Homogenisator, der in Figur 5 ohne Störstellen und in Figur 6 mit Störstellen 32 versehen ist, ergibt sich im ersten Fall eine vielfache Spiegelung sämtlicher LEDs, die im Wesentlichen gleichmäßig die Austrittsfläche des Homogenisators 11 bedecken. Die einzelnen LEDs sind allerdings als solche noch im Wesentlichen erkennbar. Wenn, wie in Figur 6, Störstellen 32 anzutreten, erzeugt die zusätzliche Streuung des Lichts im Homogenisator 11 ein vollständiges Verwaschen, so dass die einzelnen Lichtquellen nicht mehr erkennbar sind. Dies geht allerdings auf Kosten eines Intensitätsverlustes durch aus dem Homogenisator 11 herausgestreutes Licht. Anstelle von Störstellen 32 kann auch die äußere Oberfläche aufgebaut sein, sodass sich Störstellen in der Totalreflexion ergeben, die einen ähnlichen Effekt haben wie eingebettete Störstellen 32.

In Figur 7a) bis d) sind wiederum Querschnitte verschiedener Ausführungsformen erfindungsgemäß einsetzbarer Homogenisatoren gezeigt. Der Homogenisator 40 gemäß Figur 7a) hat einen runden Querschnitt und ist massiv. Die Lichtleitung erfolgt in diesem Fall durch Totalreflexion an der Außenfläche. Davon unterscheidet sich der Homogenisator 42 aus Figur 7 b) lediglich in der Form, die in diesem Fall hexagonal ist. Hier konzentrieren sich Lichtverluste auf die Kanten zwischen den einzelnen Seitenflächen.

Figur 7c) zeigt ein Beispiel eines als Reflektoranordnung ausgebildeten Homogenisators 43, der sich in seinen Außenkonturen nicht von derjenigen aus Figur 7b) unterscheidet. Innen ist er jedoch hohl und weist um seinen Innenraum 44 herum verspiegelte Innenflächen 45 auf. Figur 7d) schließlich betrifft wiederum einen massiven Homogenisator 46, der im Unterschied zu dem Ausführungsbeispiel der Figur 7a) im Zentrum einen höheren Brechungsindex aufweist als am Rand, sodass sich eine Lichtleitung durch Beugung aufgrund des Brechungsindexgradienten ergibt. An der Grenzfläche zur Umgebungsluft findet auch eine Totalreflexion statt.

In Figur 8 ist das Prinzip der Streckenaufteilung bei MQW-LED noch einmal schematisch verdeutlicht. Wiederum ist auf der horizontalen Achse eine Wellenlänge in willkürlichen Einheiten aufgetragen, auf der vertikalen Achse jeweils eine Absorptions-, Transmission- oder eine Emissionsamplitude in willkürlichen Einheiten angegeben. Mit dem Bezugszeichen 50 ist das Absorptionsspektrum eines Messgases mit einer vergleichsweise steilen Flanke bezeichnet. Das Emissionsspektrum 51 einer MQW-LED, beispielsweise der LED6 aus Figur 1, ist so gewählt, dass es nur teilweise mit der abfallenden Flanke des Absetzungsspektrums 50 überlappt. Entsprechend ist mit einer gestrichelten Linie 52 eine Wellenlängencharakteristik eines interferometrischen Strahlteilers mit steiler Flanke eingezeichnet, dessen Flanke im Wesentlichen das Emissionsspektrum 51 in zwei Teile aufteilt, die zu unterschiedlichen Messempfängern, beispielsweise Fotodioden, weitergeleitet werden. Diese beiden Anteile sind unterschiedlich schraffiert und mit dem Bezugszeichen 53 und 54 für einen Signalanteil und einen Referenzanteil bezeichnet. Der Signalanteil 43 hat einen starken Überlapp mit der abfallenden Flanke des Absorptionsspektrum 50, während der Referenzanteil 54 kaum einen Überlapp damit hat. Diese Anteile 53 und 54 sind in Figur 1a) und Figur 1b) als LED6a und LED6b bezeichnet und getrennt voneinander dargestellt, auch wenn sie aus einer einzigen Lichtquelle stammen. Statt des gezeigten Kurzpassfilters kann auch ein Bandpassfilter eingesetzt werden.

### Bezugszeichenliste

- 1: Lichtquellengruppe mit MQW-LED
- 2: spektraler Strahlteiler
- 3: Messzelle
- 4: spektraler Strahlteiler
- 5: Messempfänger
- 6: Gaseinlass
- 7: Gasauslass
- 10, 10': Lichtquellengruppe
- 11: Homogenisator
- 12: Strahlteiler
- 13: Referenz-Messempfänger
- 14: Messempfänger
- 20: Druck- und/oder Temperaturmesseinrichtung
- 21-25: Homogenisator
- 30: Abstrahlkegel
- 31: Abstrahlwinkel
- 32: Störstellen
- 34, 35: Intensitätsverteilung ausgangs des Homogenisators
- 41: homogener runder Homogenisator
- 42: homogener hexagonaler Homogenisator
- 43: innenverspiegelter Homogenisator
- 44: Hohlraum
- 45: Innenverspiegelung
- 46: Homogenisator mit Brechungsindexgradient
- 50: Absorptionsspektrum eines Gases
- 51: Emissionsspektrum einer MWQ-LED
- 52: Wellenlängencharakteristik eines interferometrischen Strahlteilers
- 53: Signalanteil
- 54: Referenzanteil

## Patentansprüche

1. Anordnung zum Messen von Gaskonzentrationen in einem Gasabsorptionsverfahren, in welchem Licht von Lichtquellen verschiedener Wellenlängen im sichtbaren, UV- und/oder IR-Bereich, insbesondere LED-Lichtquellen, durch eine Messzelle (3) mit einer zu analysierenden Gasmischung geleitet wird, insbesondere einer Mischung von NO₂, SO₂ und/oder NO in Luft, und Gaskonzentrationen zu messender Gase der Gasmischung über eine Messung einer Abschwächung des in die Messzelle (3) eingeleiteten Lichts bei verschiedenen Wellenlängen aufgrund von Absorption in den verschiedenen Gasen der Gasmischung bestimmt werden,
wobei die Anordnung eine schmale, langgestreckte, insbesondere zylindrische, Messzelle (3) umfasst, die an ihren beiden Enden jeweils Öffnungen zum Durchtritt von Licht in einen längserstreckten schmalen Strahlengang der Messzelle (3) aufweist, wobei bei einem eingangsseitigen Öffnungsdurchmesser *B* und einer Absorptionslänge *L* des Strahlengangs in der Messzelle (3) *L* > 8 gilt, insbesondere L > *5·B*, insbesondere L > *10·B,* wobei die Messzelle (3) ferner einen Gaseinlass (6) und einen Gasauslass (7) aufweist,
wobei die Anordnung weiter mehrere Lichtquellen mit unterschiedlichen Wellenlängenspektren, deren unterschiedliche Wellenlängenspektren auf Absorptionsbänder, Absorptionslücken und/oder Übergangsbereiche zwischen Absorptionsbändern und Absorptionslücken der zu messenden Gase abgestimmt sind, und mehrere Messempfänger (5, 13, 14) umfasst, mittels deren Lichtintensitäten bei mehreren der eingestrahlten Wellenlängen ausgangs der Messzelle (3) messbar sind, wobei die Anordnung zwei Messpfade umfasst,
wobei ein erster der zwei Messpfade eine erste Lichtquellengruppe (10, 10') der mehreren Lichtquellen mit unterschiedlichen Wellenlängenspektren und ein zweiter der zwei Messpfade eine zweite Lichtquellengruppe (1) der mehreren Lichtquellen mit unterschiedlichen Wellenlängenspektren aufweist
wobei die erste Lichtquellengruppe (10, 10') mehrere der Lichtquellen umfasst und ein optischer Homogenisator (11), der das Licht der verschiedenen Lichtquellen der ersten Lichtquellengruppe (10, 10')
sammelt, ihre Herkunftsorte gleichmäßig mischt und das Licht gleichmäßig abstrahlt, und der den vom durchgelassenen Licht eingenommenen Phasenraum an einen Phasenraum des Strahlengangs der Messzelle (3) anpasst, zwischen die erste Lichtquellengruppe (10, 10') und die Messzelle (3) zwischengeschaltet und direkt oder über eine gemeinsame optische Baugruppe an die Lichtquellengruppe (10, 10') angekoppelt ist, wobei die zweite Lichtquellengruppe (1) eine oder mehrere weitere der Lichtquellen umfasst,
wobei die erste Lichtquellengruppe (10, 10') und die zweite Lichtquellengruppe (1) so angeordnet sind, dass
das Licht der ersten Lichtquellengruppe (10, 10') und das Licht der zweiten Lichtquellengruppe (1) in zueinander entgegengesetzten Richtungen durch die Messzelle (3) geleitet wird, und das aus der Messzelle (3) austretende Licht der beiden Messpfade jeweils durch, insbesondere wellenlängenselektive, Strahlteiler (2, 4) der Anordnung jeweils ausgangs der Messzelle (3) zu entsprechenden Messempfängern der Messempfänger (5, 13, 14) geleitet wird, mittels deren eine Lichtintensität bei einer oder mehreren der eingestrahlten Wellenlängen ausgangs der Messzelle (13) messbar ist,
wobei die Anordnung ferner eine Auswertevorrichtung, die ausgebildet ist, aus den gemessenen Lichtintensitäten die Gaskonzentrationen zu bestimmen, umfasst.

2. Anordnung nach Anspruch 1, wobei die erste Lichtquellengruppe (10, 10') LED-Lichtquellen mit einem charakteristischen Abstrahlwinkel (31) umfasst, und die erste Lichtquellengruppe (10, 10') so vor dem Homogenisator (11) angeordnet ist, dass ein Abstrahlkegel (30) der LED-Lichtquellen der ersten Lichtquellengruppe (10, 10'), gegebenenfalls nach Durchtritt durch die gemeinsame optische Baugruppe, im Wesentlichen vollständig in den Homogenisator (11) eintritt.

3. Anordnung nach Anspruch 1 oder 2, wobei der Homogenisator (11) als ein geformter transparenter massiver Lichtleiter (41, 42, 46) auf der Grundlage von Totalreflexion an der Oberfläche oder von Brechzahlgradienten im Substrat, oder als hohle Reflektoranordnung (43) mit einem transparenten Medium im Inneren, insbesondere einem transparenten Gas oder Vakuum, und verspiegelten seitlichen Begrenzungsflächen (45) ausgebildet ist, wobei der Homogenisator (11) linear oder gebogen mit kreisrundem, ovalem oder polygonalem Querschnitt ausgebildet ist.

4. Anordnung nach Anspruch 3, wobei der Homogenisator (11) sich im Querschnitt in Richtung zur Messzelle (3) hin verändert, insbesondere verjüngt

5. Anordnung nach einem der Ansprüche 1 bis 4, wobei Störstellen (32) im oder am Homogenisator (11) angeordnet sind, insbesondere Fehlstellen im Substrat, Streukörper in einem Spiegelhohlraum oder Rauigkeiten an Grenzflächen oder Spiegelflächen.

6. Anordnung nach einem der Ansprüche 1 bis 5, wobei die Messzelle (3) an ihrer Eingangsöffnung und/oder ein an die Messzelle (3) angrenzendes Element der Anordnung ein kombiniertes Lichteintritts- und -austrittsfenster und eine gegenüber dem Lichteintritts- und -austrittsfenster
liegende lichtreflektierende Wand aufweist oder, alternativ, jeweils ein Lichteintrittsfenster und ein Lichtaustrittsfenster, mit oder ohne spiegelnde Wände zwischen dem Lichteintrittsfenster und dem Lichtaustrittsfenster aufweist, wobei das oder die Lichteintrittsfenstei und/oder Lichtaustrittsfenster insbesondere gegenüber einer Längserstreckung der Messzelle (3) schräg gestellt ist oder sind.

7. Anordnung nach einem der Ansprüche 1 bis 6, wobei eingangs und/oder ausgangs der Messzelle (3) ein oder mehrere insbesondere wellenlängenselektive
Strahlteiler (2, 4) angeordnet ist oder sind, mit dem oder mit denen Licht unterschiedlicher Lichtquellen der ersten Lichtquellengruppe (10, 10') und/oder einer zweiten Lichtquellengruppe (1) zu zwei oder mehr verschiedenen Messempfängern (5, 14) geleitet wird.

8. Anordnung nach einem der Ansprüche 1 bis 7, wobei die beiden Messpfade in einem Messvolumen der Messzelle (3) teilweise oder ganz überlappen.

9. Anordnung nach einem der Ansprüche 1 bis 8, wobei beide Lichtquellengruppen (1, 10, 10`) zusammen mit dem jeweils dazu gehörenden Messpfad und der Messzelle (3) als Anordnungen gemäß einem der Ansprüche 1 bis 7 ausgebildet sind, wobei die Messzelle (3) beiden Anordnungen gemeinsam ist.

10. Anordnung nach einem der Ansprüche 1 bis 8, wobei die Strahlteiler (2, 4) der Anordnung einen wellenlängenselektiven Strahlteiler (4) umfassen, und wobei
die zweite Lichtquellengruppe (1) wenigstens eine MQW-LED mit temperaturstabilem Emissionsspektrum (51) umfasst und der zur zweiten Lichtquellengruppe (1) gehörende zweite Messpfad ausgangs der Messzelle (3) den
wellenlängenselektiven Strahlteiler (4) und zwei Messempfänger (5, 13) der Messempfänger (5, 13, 14) umfasst, wobei der wellenlängenselektive Strahlteiler (4) eingerichtet ist, das
Emissionsspektrum (51) der MQW-LED in zwei oder mehr Anteile (53, 54) aufzuspalten und die Anteile (53, 54) getrennt voneinander zu den beiden Messempfängern (5, 13) zu leiten.

11. Anordnung nach Anspruch 10, wobei das Emissionsspektrum (51) der wenigstens einen MQW-LED sowie eine Wellenlängencharakteristik (52) des
wellenlängenselektiven Strahlteilers (4) so auf ein Absorptionsspektrum (50) eines zu messenden Gases abgestimmt sind, dass ein erster Anteil (53) des Emissionsspektrums (51) der MQW-LED eine höhere Absorption im Gas erfährt als ein zweiter Anteil (52).

12. Anordnung nach einem der Ansprüche 1 bis 11, wobei
die Anordnung eine Druck- und/oder Temperaturmesseinrichtung (20) umfasst, die mit der Messzelle (3) zur Messung eines Drucks und/oder einer Temperatur des
Gasgemisches in der Messzelle (3) verbunden ist, wobei die
Auswertevorrichtung ausgebildet ist, den Einfluss eines gemessenen Druckniveaus oder von Druckschwankungen und/oder der
Temperatur oder Temperaturschwankungen auf die Lichtabsorption oder die Gaskonzentrationen bei der Bestimmung der
Gaskonzentrationen zu berücksichtigen, gegebenenfalls auf einen
Normaldruck und/oder eine Normaltemperatur zu extrapolieren.

13. Anordnung nach einem der Ansprüche 1 bis 12, wobei die Anordnung zusätzliche Lichtleiter umfasst und wobei eine Lichteinkopplung in die Messzelle (3) und/oder eine Lichtauskopplung aus der Messzelle (3) unter Verwendung der zusätzlichen Lichtleiter erfolgt.

14. Anordnung nach einem der Ansprüche 1 bis 13, wobei die Messzelle (3) herausnehmbar ist

## Claims

1. Arrangement for measuring gas concentrations in a gas absorption method in which light from light sources of various wavelengths in the visible region, the UV region and/or IR region, especially LED light sources, is conducted through a measuring cell (3) with a gas mixture to be analysed, in particular a mixture of NO₂, SO₂ and/or NO in air, and gas concentrations of gases of the gas mixture to be measured are determined via a measurement of an attenuation of the light conducted into the measuring cell (3) at various wavelengths due to absorption in the various gases of the gas mixture,
wherein the arrangement comprises a narrow, longitudinallyextended, in particular cylindrical, measuring cell (3) having openings at each of its two ends for letting pass through light into a longitudinally-extended narrow beam path of the measuring cell (3), with an entrance-side opening diameter *B* and an absorption length *L*, with *L* > *B*, in particular *L* > *5·B,* in particular *L* > *10·B,* wherein the measuring cell (3) further has a gas inlet (6) and a gas outlet (7),
wherein the arrangement further comprises a plurality of light sources having different wavelength spectra, the different wavelength spectra of which are adjusted to absorption bands, absorption gaps and/or transition regions between absorption bands and absorption gaps of the gases to be measured, a measuring cell (3), and a plurality of measuring receivers (5, 13, 14), by means of which light intensities in a plurality of the wavelengths emitted in can be measured at the exit of the measuring cell (13),
wherein the arrangement comprises two measuring paths,
wherein a first of the two measuring paths has a first light source group (10, 10') of the plurality of light sources having different wavelength spectra and a second of the two measuring paths has a second light source group (1) of the plurality of light sources having different wavelength spectra,
wherein the first light source group (10, 10') comprises a plurality of the light sources, and an optical homogenizer (11), which collects the light from the various light sources of the first light source group (10, 10'), uniformly mixes their points of origin, and uniformly radiates the light, and which matches the phase space occupied by the transmitted light to a phase space of the optical path of the measuring cell (3), is interposed between the first light source group (10, 10') and the measuring cell (3) and is coupled to the light source group (10, 10') directly or via a common optical assembly,
wherein the first light source group (10, 10') and the second light source group (1) are arranged such that the light of the first light source group (10, 10') and the light of the second light source group (1) are guided through the measuring cell (3) in mutually opposite directions, and the light of the two measuring paths emerging from the measuring cell (3) is guided in each case through, in particular wavelength-selective, beam splitters (2, 4) of the arrangement, in each case at the outlet of the measuring cell (3), to corresponding measuring receivers of the measuring receivers (5, 13, 14), by means of which a light intensity at one or more of the irradiated wavelengths can be measured at the outlet of the measuring cell (13),
the arrangement further comprising an evaluation device designed to determine the gas concentrations from the measured light intensities.

2. Arrangement according to claim 1, wherein the first light source group (10, 10') comprises LED light sources with a characteristic radiation angle (31), and the first light source group (10, 10') is arranged in front of the homogeniser (11), such that a radiation cone (30) of the LED light sources of the first light source group (10, 10`), after passing through the common optical assembly if applicable, enters the homogeniser (11) substantially complete.

3. Arrangement according to claim 1 or 2, wherein the homogeniser (11) is designed as a shaped, transparent solid light guide (41, 42, 46) on the basis of total reflection on the surface or of refractive index gradients in the substrate or as a hollow reflector arrangement (43) with a transparent medium in the interior, in particular, a transparent gas or vacuum, and reflective lateral boundary surfaces (45), wherein the homogeniser (11) is shaped linear or curved with a circular, oval or polygonal cross-section.

4. Arrangement according to claim 3, wherein the homogeniser (11) alters, in particular tapers, in the cross-section in the direction towards the measuring cell (3).

5. Arrangement according to one of claims 1 to 4, wherein defects (32) are arranged in or on the homogeniser (11), in particular, imperfections in the substrate, dispersion bodies in a mirror cavity or roughnesses on boundary surfaces or mirror surfaces.

6. Arrangement according to one of claims 1 to 5, wherein, at its entrance opening, the measuring cell (3) and/or a member of the arrangement adjacent to the measuring cell (3) has a combined light inlet and light outlet window, and, facing the light inlet window and light outlet window, a light-reflecting wall or, alternatively, a light inlet window and a light outlet window respectively, with or without reflecting walls between the light inlet window and the light outlet window, wherein the light inlet window or light inlet windows and/or light outlet window or light outlet windows, in particular is or are inclined compared to a longitudinal extension of the measuring cell (3).

7. Arrangement according to one of claims 1 to 6, wherein, at the entrance and/or at the exit of the measuring cell (3), is or are arranged one or a plurality of, in particular wavelength-selective, beam dividers (2, 4), with which light of different light sources of the first light source group (10, 10') and/or a second light source group (1) is conducted to two or more different measuring receivers (5, 14).

8. Arrangement according to one of claims 1 to 7, wherein the the two beam paths overlap partially or completely in a measuring volume of the measuring cell (3).

9. Arrangement according to one of claims 1 to 8, wherein both light source groups (1, 10, 10'), together with the beam path belonging to each of them and the measuring cell (3), are designed as arrangements according to one of claims 1 to 7, the measuring cell (3) being common to both arrangements.

10. Arrangement according to any one of claims 1 to 8, wherein beam splitters (2, 4) of the arrangement comprise a wavelength-selective beam splitter (4), and wherein the second light source group (1) comprises at least one MQW-LED with a temperature-stable emission spectrum (51) and the second measuring path belonging to the second light source group (1) comprises the wavelength-selective beam splitter (4) at the exit of the measuring cell (3) and two measuring receivers (5, 13) of the measuring receivers (5, 13, 14), wherein the wavelength-selective beam splitter (4) is arranged to split the emission spectrum (51) of the MQW-LED into two or more components (53, 54) and to guide the components (53, 54) separately from each other to the two measuring receivers (5, 13).

11. Arrangement according to claim 10, wherein the emission spectrum (51) of the at least one MQW-LED as well as a wavelength characteristic (52) of the wavelength-selective beam splitter (4) are tuned to an absorption spectrum (50) of a gas to be measured such that a first portion (53) of the emission spectrum (51) of the MQW-LED experiences a higher absorption in the gas than a second portion (52).

12. Arrangement according to one of claims 1 to 11, wherein the arrangement comprises a pressure and/or temperature measuring device (20), which is connected to the measuring cell (3) for measuring a pressure and/or a temperature of the gas mixture in the measuring cell (3), wherein the evaluation device is designed to take into account the influence of a measured pressure level or pressure fluctuations and/or the temperature or temperature fluctuations on the light absorption or the gas concentrations when determining the gas concentrations, if appropriate to extrapolate to a normal pressure and/or a normal temperature

13. Arrangement according to one of claims 1 to 12, wherein the arrangement comprises additional light guides and wherein a light coupling into the measuring cell (3) and/or a light decoupling out of the measuring cell (3) is effected using the additional light guides.

14. Arrangement according to one of claims 1 to 13, wherein the measuring cell (3) is removable.

## Revendications

1. Système pour mesurer des concentrations de gaz dans un procédé d'absorption de gaz, dans lequel de la lumière provenant de sources lumineuses de différentes longueurs d'onde dans le domaine visible, UV et/ou IR, en particulier des sources lumineuses DEL, est guidée à travers une cellule de mesure (3) avec un mélange de gaz à analyser, en particulier un mélange de NO₂, SO₂ et/ou NO dans l'air, et des concentrations de gaz à mesurer du mélange gazeux sont déterminées par une mesure d'une atténuation de la lumière introduite dans la cellule de mesure (3) à différentes longueurs d'onde en raison de l'absorption dans les différents gaz du mélange gazeux,
le système comprenant une cellule de mesure (3) étroite et allongée, en particulier cylindrique, qui présente à chacune de ses deux extrémités des ouvertures pour le passage de la lumière selon un trajet de faisceau étroit et allongé de la cellule de mesure (3), avec, pour un diamètre d'ouverture B du côté de l'entrée et une longueur d'absorption L du trajet du faisceau dans la cellule de mesure (3), L > B, en particulier L > 5 B, en particulier L > 10·B, la cellule de mesure (3) présentant en outre une entrée de gaz (6) et une sortie de gaz (7),
le système comprenant en outre plusieurs sources de lumière avec différents spectres de longueurs d'onde, dont les différents spectres de longueurs d'onde sont adaptés aux bandes d'absorption, aux lacunes d'absorption et/ou aux zones de transition entre les bandes d'absorption et les lacunes d'absorption des gaz à mesurer, et plusieurs récepteurs de mesure (5, 13, 14), au moyen desquels les intensités lumineuses sont aptes à être mesurées à plusieurs des longueurs d'onde irradiées à la sortie de la cellule de mesure (3),
le système comprenant deux chemins de mesure,
un premier des deux chemins de mesure présentant un premier groupe de sources lumineuses (10, 10') de la pluralité de sources lumineuses avec différents spectres de longueurs d'onde et un deuxième des deux chemins de mesure présentant un deuxième groupe de sources lumineuses (1) de la pluralité de sources lumineuses avec différents spectres de longueurs d'onde,
le premier groupe de sources lumineuses (10, 10') comprenant plusieurs des sources lumineuses et un homogénéisateur optique (11) qui collecte la lumière des différentes sources lumineuses du premier groupe de sources lumineuses (10, 10'), qui mélange uniformément leurs lieux d'origine et émet uniformément la lumière, et qui adapte l'espace de phase occupé par la lumière transmise à un espace de phase du trajet optique de la cellule de mesure (3), est intercalé entre le premier groupe de sources lumineuses (10, 10') et la cellule de mesure (3) et est relié au groupe de sources lumineuses (10, 10') directement ou par l'intermédiaire d'un module optique commun,
le deuxième groupe de sources lumineuses (1) comprenant une ou plusieurs autres des sources lumineuses,
le premier groupe de sources lumineuses (10, 10') et le deuxième groupe de sources lumineuses (1) étant disposés de telle sorte que la lumière du premier groupe de sources lumineuses (10, 10') et la lumière du deuxième groupe de sources lumineuses (1) sont guidées à travers la cellule de mesure (3) dans des directions opposées l'une à l'autre, et la lumière des deux chemins de mesure sortant de la cellule de mesure (3) étant respectivement guidée par des séparateurs de faisceau (2, 4) du système, en particulier de façon sélective en longueur d'onde, respectivement à la sortie de la cellule de mesure (3) vers des récepteurs de mesure correspondants des récepteurs de mesure (5, 13, 14), au moyen desquels une intensité lumineuse est apte à être mesurée pour une ou plusieurs des longueurs d'onde rayonnées à la sortie de la cellule de mesure (13),
le système comprenant en outre un dispositif d'évaluation, qui est conçu pour déterminer les concentrations de gaz à partir des intensités lumineuses mesurées.

2. Système selon la revendication 1, dans lequel le premier groupe de sources lumineuses (10, 10') comprend des sources lumineuses DEL avec un angle de rayonnement caractéristique (31), et le premier groupe de sources lumineuses (10, 10') est disposé en avant de l'homogénéisateur (11) de telle sorte qu'un cône de rayonnement (30) des sources lumineuses DEL du premier groupe de sources lumineuses (10, 10') pénètre sensiblement complètement dans l'homogénéisateur (11), le cas échéant après avoir traversé l'ensemble optique commun.

3. Système selon la revendication 1 ou la revendication 2, dans lequel l'homogénéisateur (11) est réalisé sous la forme d'un guide de lumière (41, 42, 46) massif transparent moulé basé sur une réflexion totale en surface ou sur des gradients d'indice de réfraction dans le substrat, ou sous la forme d'un système de réflecteur creux (43) avec un milieu transparent à l'intérieur de lui, en particulier un gaz transparent ou un vide, et des surfaces (45) de délimitation latérales réfléchissantes, l'homogénéisateur (11) étant réalisé de manière linéaire ou incurvée avec une section transversale circulaire, ovale ou polygonale.

4. Système selon la revendication 3, dans lequel la section transversale de l'homogénéisateur (11) se modifie en direction de la cellule de mesure (3), en particulier se rétrécit.

5. Système selon l'une des revendications 1 à 4, dans lequel des zones de perturbation (32) sont aménagées dans ou sur l'homogénéisateur (11), en particulier des défauts dans le substrat, des corps diffusants dans une cavité miroir ou des rugosités au niveau des interfaces ou des surfaces de miroir.

6. Système selon l'une des revendications 1 à 5, dans lequel la cellule de mesure (3)
présente, au niveau de son ouverture d'entrée et/ou d'un élément du système adjacent à la cellule de mesure (3), une fenêtre d'entrée et de sortie de lumière combinée et une paroi réfléchissant la lumière située en face de la fenêtre d'entrée et de sortie de lumière
ou, en variante, présente respectivement une fenêtre d'entrée de lumière et une fenêtre de sortie de lumière, avec ou sans parois réfléchissantes entre la fenêtre d'entrée de lumière et la fenêtre de sortie de lumière,
la ou les fenêtres d'entrée de lumière et/ou fenêtres de sortie de lumière étant notamment inclinées par rapport à une extension longitudinale de la cellule de mesure (3).

7. Système selon l'une des revendications 1 à 6, dans lequel un ou plusieurs diviseurs de faisceau (2, 4), en particulier sélectifs en longueur d'onde, sont agencés à l'entrée et/ou à la sortie de la cellule de mesure (3), avec lesquels la lumière de différentes sources lumineuses du premier groupe de sources lumineuses (10, 10') et/ou d'un deuxième groupe de sources lumineuses (1) est dirigée vers deux ou plusieurs récepteurs de mesure (5, 14) différents.

8. Système selon l'une des revendications 1 à 7, dans lequel les deux chemins de mesure se chevauchent partiellement ou totalement dans un volume de mesure de la cellule de mesure (3).

9. Système selon l'une des revendications 1 à 8, dans lequel les deux groupes de sources lumineuses (1, 10, 10'), avec le chemin de mesure et la cellule de mesure (3) qui leur sont respectivement associés, sont conçus comme des systèmes selon l'une des revendications 1 à 7, la cellule de mesure (3) étant commune aux deux systèmes.

10. Système selon l'une des revendications 1 à 8, dans lequel les séparateurs de faisceau (2, 4) du système comprennent un séparateur de faisceau (4) sélectif en longueur d'onde, et dans lequel le deuxième groupe de sources lumineuses (1) comprend au moins une DEL MQW avec un spectre d'émission (51) stable en température et le deuxième chemin de mesure appartenant au deuxième groupe de sources lumineuses (1) comprend, à la sortie de la cellule de mesure (3), le séparateur de faisceau (4) sélectif en longueur d'onde et deux récepteurs de mesure (5, 13) des récepteurs de mesure (5, 13, 14), le diviseur de faisceau sélectif en longueur d'onde (4) étant agencé de façon à diviser le spectre d'émission (51) de la DEL MQW en deux ou plusieurs parties (53, 54) et pour diriger les parties (53, 54) séparément les unes des autres vers les deux récepteurs de mesure (5, 13).

11. Système selon la revendication 10, dans lequel le spectre d'émission (51) de ladite au moins une DEL MQW ainsi qu'une caractéristique de longueur d'onde (52) du diviseur de faisceau (4) sélectif en longueur d'onde sont adaptés à un spectre d'absorption (50) d'un gaz à mesurer de telle sorte qu'une première partie (53) du spectre d'émission (51) de la DEL MQW subit une absorption plus élevée dans le gaz qu'une deuxième partie (52).

12. Système selon l'une des revendications 1 à 11, dans lequel le système comprend un système (20) de mesure de la pression et/ou de la température qui est relié à la cellule de mesure (3) pour mesurer une pression et/ou une température du mélange gazeux de la cellule de mesure (3), le dispositif d'évaluation étant conçu de façon à tenir compte de l'influence d'un niveau de pression mesuré ou de variations de pression et/ou de la température ou de variations de température sur l'absorption de lumière ou les concentrations de gaz lors de la détermination des concentrations de gaz, le cas échéant pour extrapoler une pression normale et/ou une température normale.

13. Système selon l'une des revendications 1 à 12, dans lequel le système comprend des guides de lumière supplémentaires et dans lequel un couplage de lumière dans la cellule de mesure (3) et/ou un découplage de lumière de la cellule de mesure (3) est effectué en utilisant les guides de lumière supplémentaires.

14. Système selon l'une des revendications 1 à 13, dans lequel la cellule de mesure (3) est amovible.
